# EUROPÄISCHE PATENTSCHRIFT

(11) **EP 0 410 237 B1**
(45) Veröffentlichungstag und Bekanntmachung des Hinweises auf die Patenterteilung: **10.11.1993**
(21) Anmeldenummer: 90113502.0
(22) Anmeldetag: 14.07.1990
(51) Int. Cl.: A61F 2/38

(54) **Kniegelenk-Endoprothese**
Knee joint endoprosthesis
Endoprothèse pour l'articulation du genou

(30) Priorität: 26.07.1989 DE 8909036 U; 27.01.1990 DE 4002424
(43) Veröffentlichungstag der Anmeldung: 30.01.1991
(73) Patentinhaber: Bristol-Myers Squibb Company, New York, N.Y. 10154 (US)
(72) Erfinder: Schelhas, Klaus-Dieter, D-2800 Bremen 66 (DE)
(74) Vertreter: Eisenführ, Speiser & Partner

(56) Entgegenhaltungen:
- DE-A- 2 802 568
- DE-A- 3 529 894
- FR-A- 2 330 375
- GB-A- 2 129 306
- US-A- 4 301 553

## Beschreibung

Die Erfindung betrifft eine Kniegelenk-Endoprothese, deren Femurteil am unteren Ende eines Schafts konvex gekrümmte Kondylenschalen, und deren Tibiateil am oberen Ende eines Schafts ein Tibiaplateau aufweist, mit einem Verbindungsteil, welches mit einem Ende in einen Zwischenraum zwischen den Kondylenschalen hineinragt, mittels eines Querbolzens schwenkbar mit dem Femurteil verbunden ist und mit einem Zapfen drehbar in einer Bohrung des Tibiateils lagert, wobei die Kondylenschalen einer Rotationsfläche um eine Kondylenschalenachse angenähert sind, die in gebeugter Kniestellung oberhalb und parallel zur Achse des Querbolzens verläuft.

Aus der DE-A-2 802 568 ist eine derartige Kniegelenk-Endoprothese bekannt, die als Schlittenprothese ausgebildet ist, wobei deren beide Kufen durch einen Quersteg verbunden sind, an welchem ein Verbindungsteil schwenkbar an einer Querachse angeordnet ist. Das Verbindungsteil ist mit seinem freien Ende in einer Hülse gelagert, die axial in das Tibiateil implantiert ist. Aufgrund der Dimensionierung der Kondylenschalen und der Lage des Querbolzens findet bei einer Beugebewegung auch eine Abrollbewegung der Kondylenschalen auf dem Tibiaplateau statt, und zu diesem Zweck eine axiale, mittels eines Anschlags nach unten begrenzte Verschiebung des Verbindungsteils in der tibialen Hülse. Der bei dem natürlichen Kniegelenk in Strecklage vorhandene Versatz zwischen der Femurachse und der Tibiaachse wird bei dieser bekannten Knieprothese nicht verwirklicht, woraus bei längerer Benutzung Fehlbelastungen und Schmerzen resultieren können.

Aus der GB-A-2 129 306 ist eine Kniegelenk-Endoprothese bekannt, bei der das Femurteil ebenfalls mittels eines Gelenkteils mit dem Tibiateil verbunden ist, wobei jedoch das Gelenkteil im Femurteil über ein Kugelgelenk und im Tibiateil in einer Bohrung verschiebbar und begrenzt schwenkbar gelagert ist. Diese relativ lockere Verbindung der beiden Prothesenteile hat zur Folge, daß beim Ausführen einer Beugebewegung die Berührungsfläche zwischen den konvexen Kondylenschalen und den konkaven Stützflächen in Richtung der Beugebewegung wandert, d.h., daß die Schwenkbewegung des Femurteils durch eine zusätzliche Abrollbewegung relativ zum Tibiateil überlagert wird. Vorteilhafterweise wird also die Abroll- und die Gleitbewegung zwischen dem Femurteil und dem Tibiateil realisiert, allerdings werden hierzu Kugelgelenke mit einer lockeren, begrenzt verschwenkbaren Anlenkung im Tibiateil benötigt. Die fest, definierte Zuordnung und Relativbewegung von bekannten Achsknieprothesen wird dabei aufgegeben.

Aufgabe der Erfindung ist es demgegenüber, eine Kniegelenk-Endoprothese der eingangs genannten Art derart weiterzubilden, daß eine kombinierte Abroll- und Gleitbewegung zwischen Femurteil und Tibiateil und ein ausreichender Versatz zwischen Femurachse und Tibiaachse in einfacher Weise und bei geringem Resektionsbedarf verwirklicht wird.

Diese Aufgabe wird bei der Kniegelenk-Endoprothese der eingangs genannten Art erfindungsgemäß dadurch gelöst, daß auf dem Tibiaplateau ein Einsatz mit entsprechend Konkav gekrümmten Stützflächen für die Kondylenschalen angeordnet ist, daß der Zapfen des Verbindungsteils anschlagfrei axial verschiebbar gelagert ist, daß die Bohrung des Tibiateils gegen die Achse des Tibiaschafts nach dorsal versetzt ist, und daß der Querbolzen gegen die Achse der Bohrung des Tibiateils nach dorsal versetzt ist.

Die Vorteile der Erfindung liegen insbesondere darin, daß der Zapfen des Verbindungsteils in der Bohrung des Tibiateils anschlagfrei axial verschiebbar ist, wobei die Kondylenschalenachse - in Beugestellung des Kniegelenks - oberhalb der Querbolzenachse verläuft. Außerdem ist der notwendige dorsale Versatz der Femurachse relativ zur Tibiaachse in zwei Stufen, also teilweise im Tibiaplateau und teilweise im Verbindungsteil verwirklicht. Diese Merkmalskombination hat zur Folge, daß bei jeder Beugebewegung die Kondylenschalenachse parallel um die Querbolzenachse verschwenkt wird, also sich relativ zur Querbolzenachse entweder nach ventral (vorn) oder nach dorsal (hinten) bewegen kann. Da der Berührungspunkt zwischen den Kondylenflächen und dem Tibiateil lotrecht unter der Kondylenachse liegt, wandert also bei einer Beugebewegung dieser Berührungspunkt - entsprechend der Bewegung der Kondylenschalenachse - nach ventral oder nach dorsal, und es wird dadurch bei einer Beugebewegung auch eine Abrollbewegung des Femurteils auf dem Tibiaplateau verwirklicht. Außerdem wird der zur Nachbildung der natürlichen Verhältnisse notwendige, in Strecklage vorhandene Versatz zwischen Femurachse und Tibiaachse teilweise im Verbindungsteil und zum anderen im Tibiateil - durch die nach dorsal versetzte Lagerbohrung für das Verbindungsteil - realisiert, wodurch die versatzbedingte Drehmomentbelastung auf die einzelnen Teile der Prothese reduziert ist.

Besonders bevorzugt ist der Abstand der Kondylenschalenachse von der Querbolzenachse kleiner als der Durchmesser des Querbolzens, insbesondere sogar kleiner als der halbe Durchmesser des Querbolzens. Durch diese Dimensionierung wird eine ausreichende Abroll-Bewegungskomponete, d. h. eine ausreichende Bewegung des Berührungspunktes zwischen Femurteil und Tibiateil in ventraler/dorsaler Richtung erreicht. Die mit der Abrollbewegung zwangsweise einhergehende Hubbewegung der Querbolzenachse ist dadurch besonders gut dem natürlichen Bewegungsablauf des menschlichen Kniegelenkes angenähert.

Gemäß einer weiteren besonders bevorzugten Ausführungsform der Erfindung ist das Tibiaplateau gegen den Tibiaschaft in dorsal/ventraler Richtung geneigt, und zwar steigt das Tibiaplateau nach ventral - gegenüber einer senkrecht zum Tibiaschaft verlaufenden Referenzebene - um 3° bis 10° an. Diese Formgebung hat zur Folge, daß in der normalen Standposition, in welcher der menschliche Tibiaknochen zum Kniegelenk hin leicht nach vorn verläuft, das Tibiaplateau im wesentlichen horizontal ausgerichtet ist und daher dann zur Abstüt- zung des Femurteils - und des Körpergewichts - eine optimale Position einnimmt.

Besonders bevorzugt ist die Bohrung, welche in das Tibiateil hinein verläuft und den Zapfen des Verbindungsteils aufnimmt, senkrecht zur Ebene des Tibiaplateuas ausgerichtet. Durch diese Anordnung wird erreicht, daß beim entspannten, normalen Stehen die Zapfenachse, welche die Rotationsachse zwischen Tibiateil und Femurteil bildet, vertikal verläuft, daß das Femurteil auf dem Tibiateil funktionsgerecht positioniert wird und die Abrollbewegung des Femurteils in der natürlichen Lage erfolgt.

Bei einer bevorzugten Ausführungsform der Erfindung sind die den Stützflächen des Tibiaplateau-Einsatzes zugewandten Abschnitte der Kondylenschalen als Kreis- zylinder-Abschnitt ausgebildet wobei die Kreiszylinder-Achse die Kondylenschalenachse darstellt. Diese Form der Kondylenschalen ist besonders einfach und führt zu einem besonders einfachen Bewegungsablauf mit Abroll-Komponente und ermöglicht eine einfache Fertigung bei ausreichend genauer Formanpassung an die natürlichen, nur gering zu resezierenden Kondylen.

Besonders vorteilhaft ist es, den Tibiaschaft zu seinem freien Ende hin geringfügig nach medial abzuwinkeln, wobei bevorzugt der Winkel zwischen dem Tibiaplateau und dem Tibiaschaft in medialer Richtung etwa 84 bis 88° beträgt. Mit dieser Formgebung wird erreicht, daß in der normalen Standposition das Tibiaplateau auch in lateral-medialer Richtung horizontal verläuft, und daß dabei die in der Normalstellung natürlicherweise vorhandenen Winkel des Tibiaknochens und des Femurknochens relativ zu der Vertikalen verwirklicht werden, wodurch Fehlbelastungen der Knieprothese und Fehlbelastungen des natürlichen Knochen- und Bandapparates verhindert werden.

Besonders bevorzugt ist der Schaft des Femurteils in ventral-dorsaler Richtung, symmetrisch über dem Querbolzen angesetzt, an dem das Verbindungsteil schwenkbar lagert. Der Schaft des Femurteils ist nach dorsal geneigt und/oder gekrümmt, die Krümmung nimmt zum freien Ende des Femurschaftes hin ab. Durch diese Formgebung läßt sich der Femurschaft zwischen den natürlichen Kondylen an einer Stelle, wo ohnehin kein tragendes Knochengewebe vorhanden ist, nämlich bei den Kreuzbändern in den Femur einführen, wenn zuvor eine dem Femurschaft entsprechende Bohrung an dieser Stelle angebracht ist.

Weitere vorteilhafte Ausführungsformen der Erfindung sind durch die Merkmale der Unteransprüche gekennzeichnet.

Im folgenden wird ein Ausführungsbeispiel der Erfindung anhand der Zeichnungen näher erläutert. Es zeigen:
- Fig. 1: einen Querschnitt durch die Kniegelenk-Endoprothese von der Seite gesehen,
- Fig. 2: eine Ansicht des Tibiateils der Endoprothese gemäß Fig. 1 von ventral gesehen;
- Fig. 3: eine Aufsicht auf das Tibiateil gemäß Fig. 2;
- Fig. 4: eine vergrößerte Seitenansicht des Femurteils der Endoprothese gemäß Fig. 1;
- Fig. 5: einen Querschnitt längs der Linie V-V durch das Femurteil der Fig. 4;
- Fig. 6: einen Querschnitt längs der Linie VI-VI durch das Femurteil gemäß Fig. 4;
- Fig. 7: eine Seitenansicht des Verbindungsteils;
- Fig. 8: eine Ansicht des Verbindungsteils in Richtung des Pfeiles A;
- Fig. 9, 10, 11: eine schematische Darstellung einer Beugebewegung des Femurteils in verschieden stark gebeugter Position;
- Fig. 12: eine von dorsal gesehene Ansicht des die Kondylen enthaltenden unteren Abschnitts eines Femurknochens; und
- Fig. 13: eine Seitenansicht des unteren Abschnitts des Femurknochens gemäß Fig. 12.

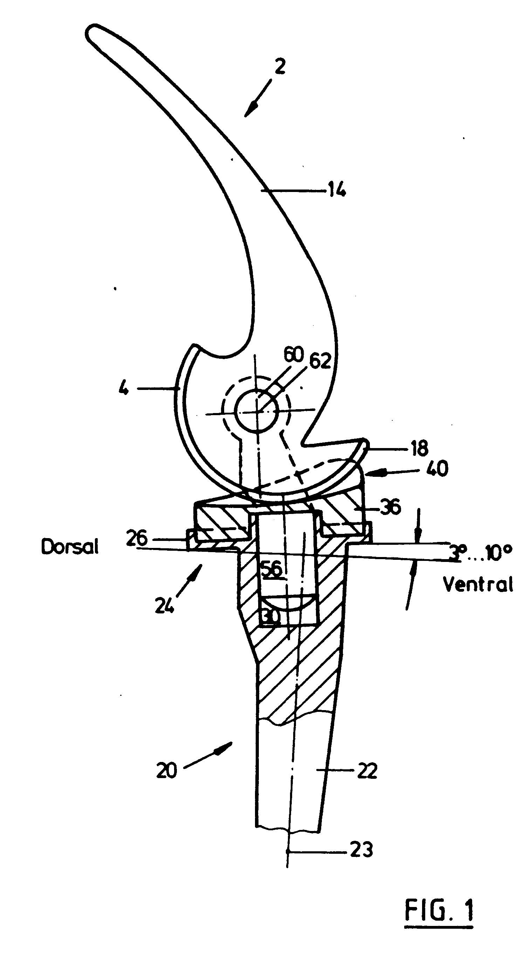

Die Kniegelenk-Endoprothese gemäß Fig. 1 besteht aus einem Femurteil 2, welches einen Schaft 14 zur Verankerung im Knochenhohlraum des menschlichen Femurknochens besitzt. Am unteren Ende des Schafts 14 sind zwei konvex gekrümmte, relativ dünnwandige Kondylenschalen 4 angeordnet, die bei einer Implantation des Femurteils 2 die Kondylen des natürlichen Femurknochen umfassen sollen.

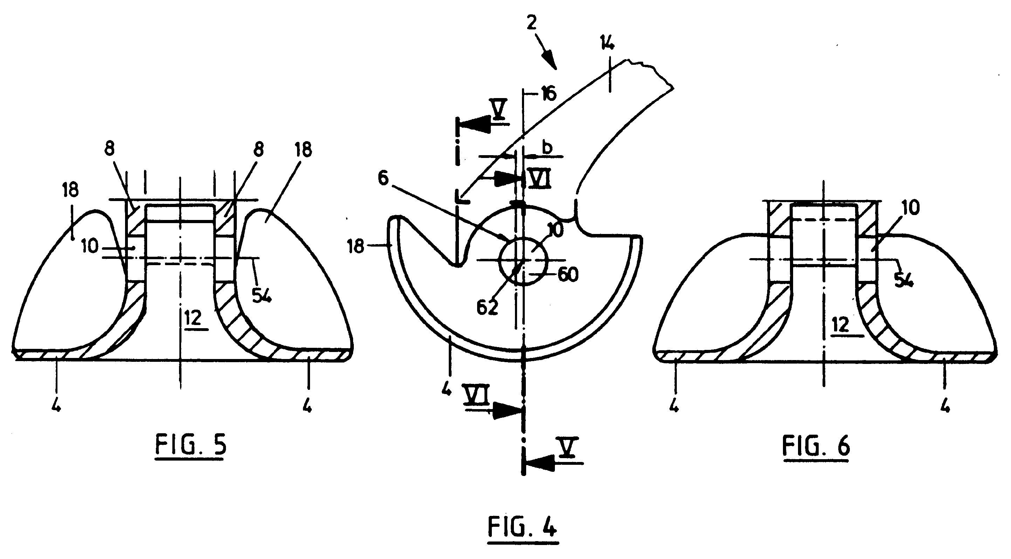

Das Femurteil 2 stützt sich über die Kondylenschalen 4 auf einem Tibiateil 20 ab, welches ein Tibiaplateau 24, und einen Einsatz 36 auf dem Tibiaplateau 24 aufweist. Der Tibiaplateau-Einsatz 36 besitzt unterhalb der Kondylenschalen 4 je eine entsprechend konkav gekrümmte Stützfläche 38 eingeformt, zwischen den Stützflächen 38 verläuft in ventral-dorsaler Richtung ein Höcker 40, der sich in einem unter dem Schaft 14 verlaufenden Zwischenraum 12 zwischen den Kondylenschalen 4 erstreckt, vgl. auch die Fig. 5 und 6, und als Anschlag für eine Rotationsbewegung des Femurteil 2 relativ zum Tibiateil 20 dient. An der Unterseite des Tibiaplateaus 24 befindet sich ein Schaft 22, der geringfügig angewinkelt vom Tibiaplateau 24 nach unten absteht und zur Verankerung des Tibiateils im Knochenhohlraum des natürlichen Tibiaknochens dient.

Das Femurteil 2 ist mit einem Verbindungsteil 50 mit dem Tibiateil 20 so gekoppelt, daß das Femurteil 2 eine Beuge- oder Schwenkbewegung auf dem Tibiaplateau 24 um eine - in Darstellung - horizontale Achse, und außerdem eine Rotationsbewegung um eine - in der Darstellung - im wesentlichen vertikale Achse ausführen kann. Am oberen Ende des Verbindungsteils 50 ist zu diesem Zweck ein Auge 52 angeformt, welches in den Zwischenraum 12 zwischen den Kondylenschalen 4 hineinragt und mittels eines Querbolzens 60 schwenkbar mit dem Femurteil 2 verbunden ist. Der Querbolzen 60 ist in Bohrungen 10 der beiden Seitenwände 8 gelagert, welche in vorgegebenem Abstand einander gegenüber liegen und zwischen sich den Zwischenraum 12 bilden, in dem das Verbindungsteil 50 auf dem Querbolzen 60 lagert. Das untere Ende des Verbindungsteils 50 ist als Zapfen 56 ausgebildet, der quer zur Schwenkachse verläuft, die mit der Achse 54 des Querbolzens 60 zusammenfällt. Der Zapfen 56 lagert in einer Bohrung 30, die senkrecht zur Ebene des Tibiaplateaus von oben in das Tibiaplateau in Richtung des Schafts 22 eingearbeitet ist.

Der Zapfen 56 des Verbindungsteils 50 ist im Tibiateil 20 anschlagfrei axial verschiebbar gelagert, so daß sich das Verbindungsteil 50 bei einer Beugebewegung des Femurteils 2 aus der Bohrung 30 heraus bzw. in die Bohrung hinein verschieben läßt.

Die den Stützflächen 38 des Tibiaplateau-Einsatzes 36 zugewandten Abschnitte der Kondylenschalen 4 sind einer Rotationsfläche um eine Kondylenschalenachse 6 angenähert, sie sind im dargestellten Ausführungsbeispiel Teil einer Kreiszylinder-Oberfläche, dessen Zylinderachse die Kondylenschalenachse 6 darstellt, die parallel zur Achse 62 des Querbolzens 6 verläuft und gegen die Achse 62 des Querbolzens 60 einen vorgegebenen Versatz a nach oben und ggf. auch geringfügig nach ventral besitzt.

Wie sich insbesondere den Fig. 4 bis 6 entnehmen läßt, ist der Abstand a der Kondylenschalenachse 6 von der Achse 62 des Querbolzens 60 in der dargestellten Ausführungsform kleiner als der halbe Durchmesser d des Querbolzens. Die Kondylenschalenachse 6 weist gegenüber einer Referenzebene 16, die durch die Achse 62 des Querbolzens und im wesentlichen senkrecht zu der Verbindungslinie von ventraler und dorsaler Endkante der Kondylenschalen 4 verläuft einen geringen Versatz b nach ventral.

Wie insbesondere in Fig. 1 bis 3 entnehmbar ist, ist das Tibiaplateau 24 des Tibiateils 20 gegen den Tibiaschaft 22 in dorsal-ventraler Richtung geneigt und steigt - gegenüber einer lotrecht auf dem Schaft 23 sitzenden Referenzebene nach ventral um 3 bis 10°, besonders bevorzugt um 4 bis 6° an. Die Bohrung 30 des Tibiateils 20 verläuft senkrecht zur Ebene des Tibiaplateaus 24 und ist gegen die Achse 23 des Tibiaschaftes 22 versetzt.

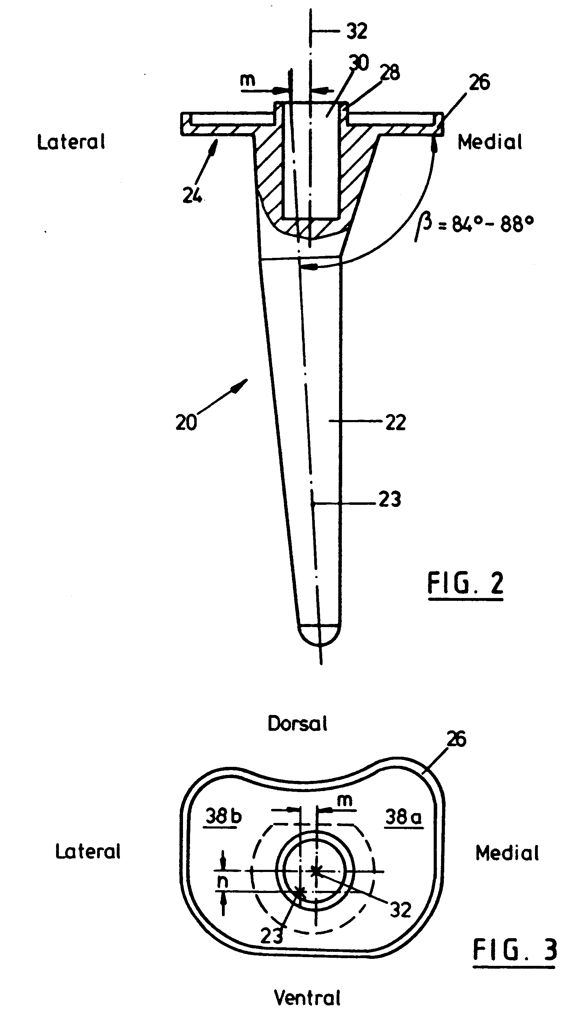

Wie insbesondere Fig. 3 entnehmbar ist, ist die Achse 32 der Bohrung 30 in der Ebene des Tibiaplateaus 24 gegen die Achse 23 des Tibiaschafts 22 nach medial um einen Abstand m, und nach dorsal um einen Abstand n versetzt. Der Tibiaschaft besitzt - von ventral gesehen - außerdem eine geringfügige Neigung nach medial. Der Winkel β , den der Tibiaschaft nach medial mit dem Tibiaplateau 24 bildet, beträgt zwischen 84 bis 88°. Durch diese Anwinkelung des Tibiaschaftes 22 wird erreicht, daß das Tibiaplateau 24 bei normaler Standposition eines Patienten auch in der lateral-medialen Richtung horizontal verläuft.

Wie den Fig. 1 bis 3 entnehmbar ist, besitzt das Tibiaplateau 24 einen umlaufenden Randwulst 26 sowie einen um die Bohrung 30 umlaufenden Randsteg 28 zur sicheren Einfassung des Tibiaplateau-Einsatzes 36.

Wie insbesondere Fig. 3 entnehmbar ist, ist die mediale Stützfläche 38a des Tibiaplateau-Einsatzes 36 größer und erstreckt sich weiter nach dorsal als die laterale Stützfläche 38b.

Die Fig. 4 bis 6 zeigen eine vergrößerte Seitenansicht und Schnitte des Femurteils 2. Gemäß Fig. 4 besitzen die Kondylenschalen 4 ventral einen Abschnitt 18 mit kleinerer Krümmung, um eine bessere Anpassung an die Form der natürlichen Kondylen zu verwirklichen. Der Schaft 14 des Femurteils ist - in ventral-dorsaler Richtung - etwa symmetrisch über dem Querbolzen 60 angesetzt, der das Verbindungsteil 50 im Zwischenraum 12 schwenkbar hält. Der Schaft 14 ist nach dorsal geneigt und/oder gekrümmt, um beim Implantieren ein einfaches Einsetzen des Femurteils von dorsal in eine Bohrung zwischen den Kondylen zu ermöglichen.

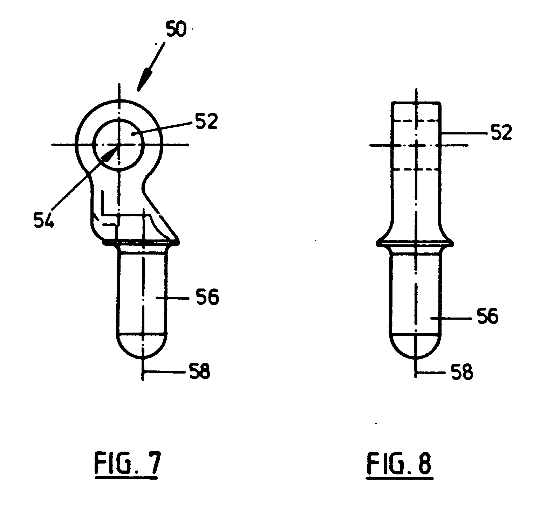

Die Fig. 7 und 8 zeigen das Verbindungsteil 50 in Seitenansicht und in Frontansicht. Wie Fig. 7 entnehmbar ist, ist die Achse 58 des Zapfens 56 gegenüber der Achse 54 des Auges 52 durch welches der Querbolzen 60 verläuft, um einen Versatz v nach ventral versetzt. Dadurch kommt die Querbolzenachse 62 gegenüber der Bohrung 30 des Tibiateils um den Versatz v nach dorsal zu liegen und nimmt dann eine der natürlichen Beugeachse entsprechende Position ein.

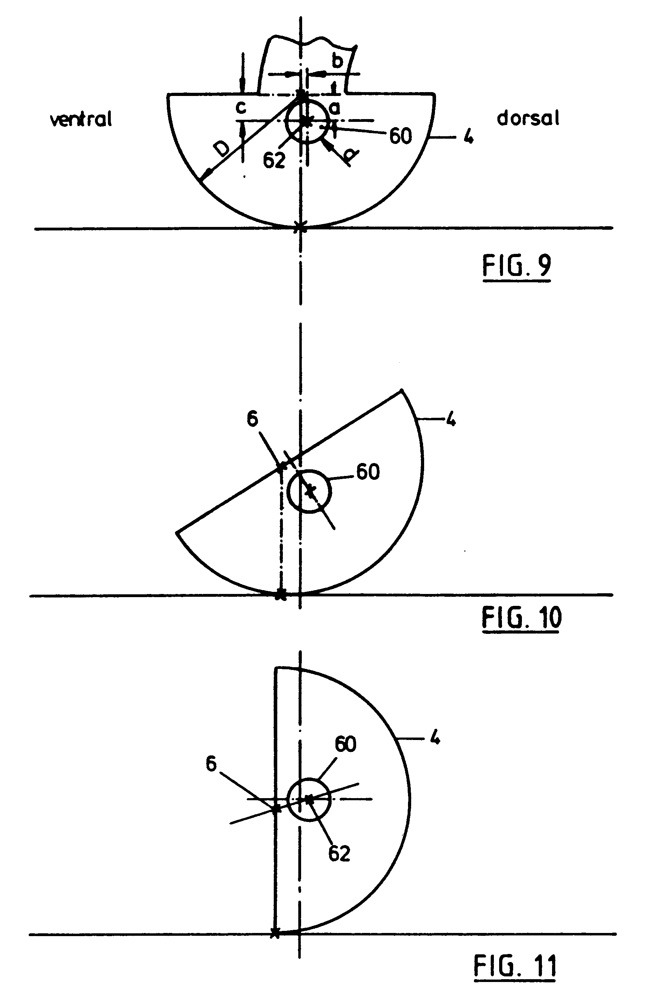

In den Fig. 9 bis 11 ist die Beugebewegung des Femurteils 2 mit verschiedenen Beugewinkeln dargestellt, der Einfachheit halber sind jedoch die Kondylenschalen als 180°-Abschnitt einer Kreiszylinderfläche gezeichnet. Die Kondylenschalenachse 6 verläuft - wie bei der erfindungsgemäßen Kniegelenk-Endoprothese - um einen vorgegebenen Versatz a über der Achse 62 des Querbolzens 60, der einen Durchmesser d besitzt. Der Durchmesser des die Kondylenschalen bildenden Kreiszylinder-Abschnitts ist mit D bezeichnet.

Fig. 9 zeigt das Femurteil 2 in einer relativ stark gebeugten oder abgewinkelten Stellung des Kniegelenks, bei der die Kondylenschalenachse 6 lotrecht über der Achse 62 des Querbolzens 60 liegt, so daß die Kondylenschalen den Tibiaplateau-Einsatz 36 ebenfalls lotrecht unter der Querbolzenachse 62 berühren.

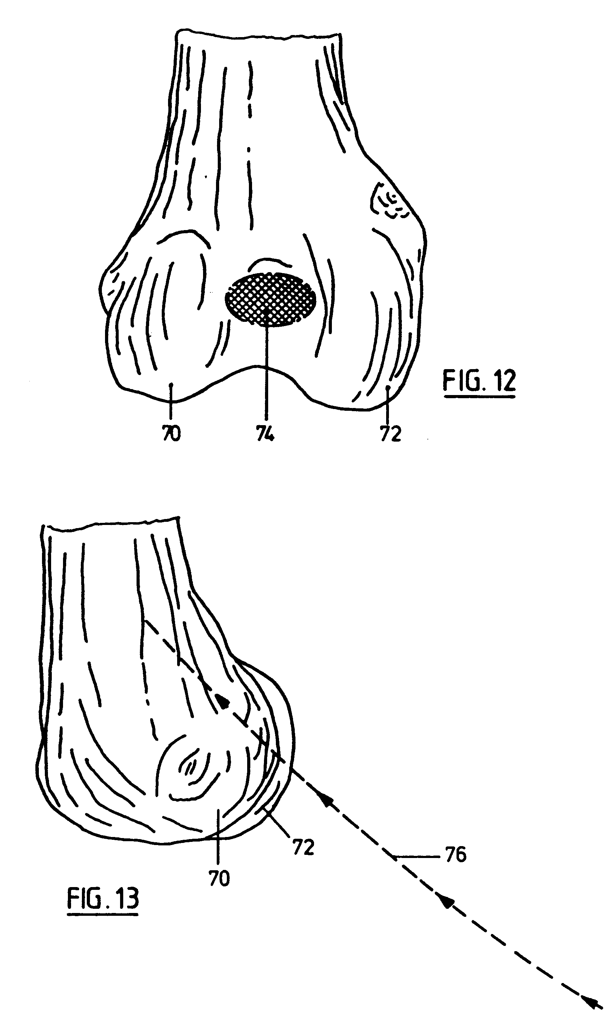

Wenn der Beugewinkel abnimmt, das Femurteil 2 sich also der Strecklage stärker annähert, vgl. Fig. 10, so wird die Kondylenschalenachse 6 dabei um die Querbolzenachse 62 nach ventral geschwenkt; der Berührungspunkt zwischen Kondylenschalen und Tibiaplateau-Einsatz liegt - wie bei einem abrollenden Kreiszylinder - stets lotrecht unter der Kondylenschalenachse 6, d. h. der Berührungspunkt zwischen Femurteil und Tibiateil "rollt" bei diesem Bewegungsablauf mit zunehmendem Übergang in die Strecklage - nach ventral. Bei vollständiger Streckung, vgl. Fig. 11, liegt der Berührungspunkt zwischen Femurteil und Tibiateil um den Versatz a ventral vor der Querbolzenachse 62. Mit zunehmendem Übergang in die Strecklage wandert daher der Berührungspunkt zwischen Femurteil und Tibiateil nach ventral, auf diese Weise wird die Abroll-Bewegungskomponente der Kniegelenk-Endoprothese realisiert.

Die Implantation der Kniegelenk-Endoprothese ist außerordentlich gewebeschonend und beläßt im Gegensatz zu bisher bekannten Prothesen die wesentlichen Teile des Femurs bzw. dessen Kondylen ohne Schwächung. Da die Endoprothese keinen breiten femuralen Block besitzt, der im natürlichen Femurgewebe verankert werden muß und folglich ein entsprechend großes interkondyläres Fenster erfordert, ist die Resektion eines entsprechend großen Knochengewebeteils nicht notwendig. Vielmehr wird bei der erfindungsgemäße Endoprothese lediglich zwischen den natürlichen Kondylen 70, 72 - vgl. die Fig. 12 und 13 - an einer Stelle, wo ohnehin kein tragendes Knochengewebe vorhanden ist, nämlich bei den Kreuzbändern, eine dem Femurschaft 14 bzw. dessen Durchmessers entsprechende Bohrung 74 angebracht, wie sie in Fig. 12 mit einer strichpunktierten Elypse angedeutet und kreuzschraffiert dargestellt ist, und die Prothese wird sodann mit dem freien Endabschnitt des gekrümmten Femurschaftes 14 voran in Richtung der Pfeil-Strichlinie 76, vgl. Fig. 13, in die Bohrung 74 eingebracht. Aufgrund der Krümmung des Femurschaftes 14 läßt sich dieser ohne Schwierigkeiten durch die Bohrung 74 in den Markkanal des Femurs 3 einbringen.

Besonders bevorzugt besitzt der Tibiaplateau-Einsatz 36 auch im dorsalen Bereich zwischen den beiden Stützflächen 38a und 38b einen Höcker zur Begrenzung der Rotationsbewegung.

## Patentansprüche

1. Kniegelenk-Endoprothese, deren Femurteil (2) am unteren Ende eines Schafts (14) konvex gekrümmte Kondylenschalen (4), und deren Tibiateil (20) am oberen Ende eines Schafts (22) ein Tibiaplateau (24) aufweist,
mit einem Verbindungsteil (50), welches mit einem Ende in einen Zwischenraum (12) zwischen den Kondylenschalen (4) hineinragt, mittels eines Querbolzens (60) schwenkbar mit dem Femurteil (2) verbunden ist und mit einem Zapfen (56) drehbar in einer Bohrung (30) des Tibiateils (20) lagert, wobei die Kondylenschalen (4) einer Rotationsfläche um eine Kondylenschalenachse (6) angenähert sind, die in gebeugter Kniestellung oberhalb und parallel zur Achse (62) des Querbolzens (60) verläuft,
dadurch gekennzeichnet, daß auf dem Tibiaplateau (24) ein Einsatz (36) mit entsprechend konkav gekrümmten Stützflächen (38) für die Kondylenschalen (4) angeordnet ist, daß der Zapfen (56) des Verbindungsteils (50) anschlagfrei axial verschiebbar gelagert ist, daß die Bohrung (30) des Tibiateils (20) gegen die Achse (23) des Tibiaschafts (22) nach dorsal versetzt ist, und daß der Querbolzen (60) gegen die Achse (32) der Bohrung (30) des Tibiateils (20) nach dorsal versetzt ist.

2. Kniegelenk-Endoprothese nach Anspruch 1,
dadurch gekennzeichnet, daß der Abstand (a) der Kondylenschalenachse (6) von der Achse (62) des Querbolzens (60) kleiner ist als der Durchmesser (d) des Querbolzens (60).

3. Kniegelenk-Endoprothese nach Anspruch 2,
dadurch gekennzeichnet, daß der Abstand (a) der Kondylenschalenachse (6) von der Achse (62) des Querbolzens (60) kleiner ist als der halbe Durchmesser (d) des Querbolzens (60).

4. Kniegelenk-Endoprothese nach Anspruch 1 oder 2,
dadurch gekennzeichnet, daß das Tibiaplateau (24) gegen den Tibiaschaft (22) in dorsal-ventraler Richtung geneigt ist und nach ventral um 3 bis 10° ansteigt.

5. Kniegelenk-Endoprothese nach einem der vorstehenden Ansprüche,
dadurch gekennzeichnet, daß die Bohrung (30) senkrecht zur Ebene des Tibiaplateaus (24) von oben in das Tibiateil (20) hinein verläuft.

6. Kniegelenk-Endoprothese nach Anspruch 5,
dadurch gekennzeichnet, daß die Achse (32) der Bohrung (30) des Tibiateils (20) in der Ebene des Tibiaplateaus (24) gegen die Achse (23) des Tibiaschafts (22) nach medial versetzt ist.

7. Kniegelenk-Endoprothese nach einem der vorstehenden Ansprüche,
dadurch gekennzeichnet, daß die den Stützflächen (38) des Tibiaplateau-Einsatzes (36) zugewandten Abschnitte der Kondylenschalen (4) als Rotationsfläche einen Kreiszylinder-Abschnitt bilden.

8. Kniegelenk-Endoprothese nach einem der vorstehenden Ansprüche,
dadurch gekennzeichnet, daß die Kondylenschalen (4) ventral in einen Abschnitt (18) mit kleinerer Krümmung übergehen.

9. Kniegelenk-Endoprothese nach einem der vorstehenden Ansprüche,
dadurch gekennzeichnet, daß der Tibiaschaft (22) zu seinem freien Ende hin geringfügig nach medial geneigt verläuft.

10. Kniegelenk-Endoprothese nach Anspruch 8,
dadurch gekennzeichnet, daß der Winkel (β), den der Tibiaschaft (22) in medialer Richtung mit dem Tibiaplateau (24) bildet, etwa 84° bis 88° beträgt.

11. Kniegelenk-Endoprothese nach einem der vorstehenden Ansprüche,
dadurch gekennzeichnet, daß die mediale Stützfläche (38a) des Tibiaplateau-Einsatzes (36) sich weiter nach dorsal erstreckt als die laterale Stützfläche (38b).

12. Kniegelenk-Endoprothese nach einem der vorstehenden Ansprüche,
dadurch gekennzeichnet, daß der Schaft (14) des Femurteils (2) nach dorsal geneigt und/oder gekrümmt verläuft.

13. Kniegelenk-Endoprothese nach einem der vorstehenden Ansprüche,
dadurch gekennzeichnet, daß der Schaft (14) des Femurteils (2) in ventral-dorsaler Richtung etwa symmetrisch über dem Querbolzen (60) ansetzt.

## Claims

1. A knee-joint endoprosthesis, the femur part (2) of which at the lower end of a shaft (14) comprises convexly curved condyle shells (4), and the tibia part (20) of which at the upper end of a shaft (22) comprises a tibia plate (24), having a connecting part (50), which with one end protrudes into a space (12) between the condyle shells (4), is swivellably connected to the femur part (2) by means of a cross bolt (60) and with a peg (56) is pivoted in a bore hole (30) in the tibia part (20), whereby the condyle shells (4) are approximately a surface of revolution around a condyle shell axis (6), which in the flexed knee position extends above and parallel to the axis (62) of the cross bolt (60),
**characterised in that** an insert (36) having correspondingly curved support surfaces (38) for the condyle shells (4) is disposed on the tibia plate (24),
**in that** the peg (56) of the connecting part (50) is housed in an axially displaceably manner without impact,
**in that** the bore hole (30) in the tibia part (20) is offset on the dorsal side towards the axis (23) of the tibia shaft (22),
**and in that** the cross bolt (60) is offset on the dorsal side towards the axis (32) of the bore hole (30) in the tibia part (20).

2. A knee-joint- endoprosthesis according to Claim 1,
**characterised in that** the distance (a) of the condyle shell axis (6) from the axis (62) of the cross bolt (60) is less than the diameter (d) of the cross bolt (60).

3. A knee-joint endoprosthesis according to Claim 2,
**characterised in that** the distance (a) of the condyle shell axis (6) from the axis (62) of the cross bolt (60) is less than half the diameter (d) of the cross bolt (60).

4. A knee-joint endoprosthesis according to Claim 1 or 2,
**characterised in that** the tibia plate (24) is inclined in the dorsal-ventral direction towards the tibia shaft (22) and rises towards the ventral side by 3 to 10°.

5. A knee-joint endoprosthesis according to one of the preceding Claims,
**characterised in that** the bore hole (30) extends from above into the tibia part (20) perpendicular to the plane of the tibia plate (24).

6. A knee-joint endoprosthesis according to Claim 5,
**characterised in that** the axis (32) of the bore hole (30) of the tibia part (20) in the plane of the tibia plate (24) is displaced in the medial direction towards the axis (23) of the tibia shaft (22).

7. A knee-joint prosthesis according to one of the preceding Claims,
**characterised in that** the sections of the condyle shells (4) close to the support surfaces (38) of the tibia plate insert (36) as a surface of revolution form a regular cylinder section.

8. A knee-joint endoprosthesis according to one of the preceding Claims,
**characterised in that** at the ventral side the condyle shells (4) pass into a section (18) having less curvature.

9. A knee-joint endoprosthesis according to one of the preceding Claims,
**characterised in that** the tibia shaft (22) extends inclined slightly in the medial direction towards its free end.

10. A knee-joint endoprosthesis according to Claim 8,
**characterised in that** the angle (β), which the tibia shaft (22) forms in the medial direction with the tibia plate (24), is roughly 84° to 88°.

11. A knee-joint endoprosthesis according to one of the preceding Claims,
**characterised in that** the medial support surface (38a) of the tibia plate insert (36) extends further in the dorsal direction than the lateral support surface (38b).

12. A knee-joint endoprosthesis according to one of the preceding Claims,
**characterised in that** the shaft (14) of the femur part (2) extends inclined and/or curved towards the dorsal side.

13. A knee-joint endoprosthesis according to one of the preceding Claims,
**characterised in that** the shaft (14) of the femur part (2) is positioned roughly symmetrically above the cross bolt (60) in the ventral-dorsal direction.

## Revendications

1. Endoprothèse pour l'articulation du genou, dont la partie formant fémur (2) présente à l'extrémité inférieure d'une tige (14) des coques formant condyle incurvées de manière convexe, et dont la partie formant tibia (20) présente un plateau de tibia (24) à l'extrémité supérieure d'une tige (22), avec une piece de liaison (50), laquelle pénètre avec une extrémité dans un interstice (12) entre les coques formant condyle (4), est reliée au moyen d'un boulon transversal (60) avec la partie formant fémur (2), de manière à pouvoir pivoter, et repose en rotation, avec une cheville, dans un trou (30) de la partie formant tibia (20), les coques formant condyle (4) étant rapprochées d'une surface de rotation autour d'un axe de coque formant condyle (6), qui s'étend, lorsque le genou est en position pliée, au-dessus de et parallèlement à l'axe (62) du boulon transversal (60), caractérisée en ce qu'un insert (36) avec des surfaces d'appui (38) incurvées d'une manière correspondante, concave, pour les coques formant condyles (4), est disposé sur le plateau de tibia (24), que la cheville (56) de la pièce de liaison (50) est supportée sans butée, de manière à pouvoir se déplacer dans le sens axial, que le trou (30) de la partie formant tibia (20) est décalée vers la position dorsale, vers l'axe (23) de la tige de tibia (22), et que le boulon transversal (60) est décalé vers la position dorsale vers l'axe (32) du trou (30) de la partie formant tibia (20).

2. Endoprothèse pour l'articulation du genou selon la revendication 1, caractérisée en ce que l'écartement (a) de la coque formant condyle (6) à l'axe (62) du boulon transversal (60) est plus petit que le diamètre (d) du boulon transversal (60).

3. Endoprothèse pour l'articulation du genou selon la revendication 2, caractérisée en ce que l'écartement (a) de la coque formant condyle (6) à l'axe (62) du boulon transversal (60) est plus petit que la moitié du diamètre (d) du boulon transversal (60).

4. Endoprothèse pour l'articulation du genou selon l'une quelconque des revendications 1 ou 2, caractérisée en ce que le plateau de tibia (24) est incliné vers la tige de tibia (22) dans le sens dorsal-ventral et s'élève de 3 à 10° vers la position ventrale.

5. Endoprothèse pour l'articulation du genou selon l'une quelconque des revendications précédentes, caractérisée en ce que le trou (30) s'étend perpendiculairement au plan du plateau de tibia (24) depuis le haut dans la partie formant tibia (20).

6. Endoprothèse pour l'articulation du genou selon la revendication 5, caractérisée en ce que l'axe (32) du trou (30) de la partie formant tibia (20) est décalée dans le plan du plateau de tibia (24) vers l'axe de la tige de tibia (22), vers la position médiane.

7. Endoprothèse pour l'articulation du genou selon l'une quelconque des revendications précédentes, caractérisée en ce que les segments des coques formant condyle (4) orientés vers les surfaces d'appui (38) de l'insert formant plateau de tibia (36), constituent en tant que surface de rotation un segment de cylindre circulaire.

8. Endoprothèse pour l'articulation du genou selon l'une quelconque des revendications précédentes, caractérisée en ce que les coques formant condyle (4) dans la position ventrale se transforment un segment d'une courbure plus petite.

9. Endoprothèse pour l'articulation du genou selon l'une quelconque des revendications précédentes, caractérisée en ce que la tige de tibia (22) est orientée légèrement vers la position médiane, dans la direction de son extrémité libre.

10. Endoprothèse pour l'articulation du genou selon la revendication 8, caractérisée en ce que l'angle (β), que constitue la tige de tibia (22) dans la direction médiane avec le plateau de tibia (24), est compris environ entre 84° et 88°.

11. Endoprothèse pour l'articulation du genou selon l'une quelconque des revendications précédentes, caractérisée en ce que la surface d'appui médiane (38a) de l'insert formant plateau de tibia (36) s'étend plus loin dans la direction dorsale que la surface d'appui latérale (38b).

12. Endoprothèse pour l'articulation du genou selon l'une quelconque des revendications précédentes, caractérisée en ce que la tige (14) de la partie formant fémur (2) s'étend inclinée et/ou incurvée vers la position dorsale.

13. Endoprothèse pour l'articulation du genou selon l'une quelconque des revendications précédentes caractérisée en ce que la tige (14) de la partie formant fémur (2) fait saillie dans le sens ventral-dorsal sensiblement symétriquement au-dessus du boulon transversal (60).
